# NEUE EUROPÄISCHE PATENTSCHRIFT

(11) **EP 1 000 244 B2**
(45) Veröffentlichungstag und Bekanntmachung des Hinweises auf die Entscheidung über den Einspruch: **31.10.2007**
(45) Hinweis auf die Patenterteilung: 11.09.2002
(21) Anmeldenummer: 98943745.4
(22) Anmeldetag: 03.07.1998
(51) Int. Cl.: F04B 37/14, F04B 37/20, A61L 2/06, F04B 39/10, F04B 39/06

(54) **VERFAHREN ZUM EVAKUIEREN EINES FEUCHTEN GASES, BEARBEITUNGSVORRICHTUNG ZUR DURCHFÜHRUNG DIESES VERFAHRENS SOWIE SAUGPUMPE FÜR EINE SOLCHE BEARBEITUNGSVORRICHTUNG**
METHOD FOR EVACUATING A WET GAS, TREATMENT DEVICE FOR CARRYING OUT THIS METHOD AND SUCTION PUMP FOR A TREATMENT DEVICE OF THIS TYPE
PROCEDE POUR L'EVACUATION D'UN GAZ HUMIDE, DISPOSITIF DE TRAITEMENT POUR LA MISE EN OEUVRE DE CE PRODEDE, AINSI QUE POMPE D'ASPIRATION DESTINEE A UN TEL DISPOSITIF DE TRAITEMENT

(30) Priorität: 30.07.1997 DE 19732808
(43) Veröffentlichungstag der Anmeldung: 17.05.2000
(60) Teilanmeldung: 01112768.5
(73) Patentinhaber: KNF Neuberger GmbH, D-79112 Freiburg (DE)
(72) Erfinder: BECKER, Erich, D-79189 Bad Krozingen (DE); RIEDLINGER, Heinz, D-28211 Bremen (DE)
(74) Vertreter: Patent- und Rechtsanwaltssozietät Maucher, Börjes & Kollegen
(86) Internationale Anmeldenummer: PCT/EP1998/004509
(87) Internationale Veröffentlichungsnummer: WO 1999/006699

(56) Entgegenhaltungen:
- WO-A-96/12557
- DE-A- 2 162 031
- DE-A- 3 533 017
- DE-A- 3 710 782
- DE-A- 4 215 090
- DE-A- 4 445 054
- DE-C- 945 286
- US-A- 3 027 651
- US-A- 4 113 410
- US-A- 4 580 604
- US-A- 5 603 611
- Lexikon der Verfahrenstechnik Seite 528
- Technische Information über Elektromagnetische Dosierpumpe
- Technische Information zu Dosier-Membranpumpe
- Artikel zu Miele-Pumpenkopf
- Ersatzteilliste Pumpenkopf
- Dubbel Taschenbuch für den Maschinenbau Seiten P24, P25
- Technische Information zu Dosierpumpenprinzip

## Beschreibung

Die Erfindung betrifft ein Verfahren zum Evakuieren eines feuchten oder flüssigen Fördermediums aus der Bearbeitungskammer einer Bearbeitungsvorrichtung mittels einer Fördereinrichtung, die eine ein- oder mehrstufige Saugpumpe hat, wobei das Fördermedium während der Evakuierung im Verlauf des Strömungsweges derart gekühlt wird, daß sich das Fördermedium in der Fördereinrichtung in flüssigem Aggregatzustand befindet oder überführt wird.

Die Erfindung befaßt sich auch mit einer Bearbeitungsvorrichtung zur Durchführung des eingangs erwähnten Verfahrens mit einer Bearbeitungskammer, welche zur Evakuierung an eine Fördereinrichtung angeschlossen ist, sowie mit einer ein- oder mehrstufigen Saugpumpe, die der Fördereinrichtung zugeordnet ist, wobei die Fördereinrichtung zumindest in einem Teilbereich mittels wenigstens einer Kühleinrichtung derart kühlbar ist, daß sich das Fördermedium in der Fördereinrichtung in flüssigem Aggregatzustand befindet oder überführt wird.

Darüber hinaus beinhaltet die Erfindung auch eine ein- oder mehrstufige Membranpumpe für die oben beschriebene Bearbeitungsvorrichtung mit wenigstens einem, in einem Pumpenkopf befindlichen Einlaß- und zumindest einem Auslaßventil, von denen zumindest ein Ventil eine vom Fördermedium gesteuerte Ventilscheibe aufweist, die in Schließstellung des Ventils an einer, zumindest eine Ventilöffnung umgrenzenden Ventil-Dichtfläche des Pumpenkopfes dichtend anliegt.

Derartige Bearbeitungsvorrichtungen sind beispielsweise als Autoklaven zum Sterilisieren medizinischer Gebrauchsgegenstände bereits bekannt. Diese vorbekannten Bearbeitungsvorrichtungen weisen eine luftdicht verschließbare Bearbeitungskammer auf, in welche die darin befindlichen Instrumente vor dem Sterilisationsvorgang zunächst einem sogenannten fraktionierten Vorvakuum ausgesetzt werden, bei dem durch wiederholtes Evakuieren der Luft im Wechsel mit dem periodischen Einströmen von Dampf eine besonders gute Luftentfernung auch aus englumigen Instrumenten erreicht wird. Während des Sterilisationsvorganges werden die Instrumente in der Bearbeitungs- oder Sterilisationskammer unter Überdruck heißem Wasserdampf ausgesetzt. Um die Instrumente nach dem Sterilisieren rasch und rückstandslos zu trocknen, wird in der Bearbeitungskammer anschließend wiederum ein sogenanntes Nachvakuum erzeugt, welches die Trocknungszeit des Sterilisiergutes verkürzen und den Trocknungsvorgang optimieren soll.

Zum Evakuieren ist die Sterilisationskammer solch vorbekannter Dampfsterilisationsvorrichtungen an eine Fördereinrichtung angeschlossen, die eine Vakuumpumpe aufweist. Wegen der Beaufschlagung der Vakuumpumpe mit Wasserdampf werden bislang nur Wasserringpumpen oder Membranpumpen verwendet. Wegen der Baugröße und der Nachteile einer Wasserringpumpe kommen in den beispielsweise für die Arztpraxis vorgesehenen kleineren Dampfsterilisationsvorrichtungen meist nur Membranpumpen in Frage.

Man hat daher auch bereits eine als Dampfsterilisator ausgebildete Bearbeitungsvorrichtung der eingangs erwähnten Art geschaffen, die zur Evakuierung ihrer Bearbeitungs- oder Sterilisationskammer eine Fördereinrichtung hat, welcher eine wasserfrei betriebene Saugpumpe zugeordnet ist, wobei in der zwischen Bearbeitungskammer und Saugpumpe vorgesehenen Saugleitung ein wasserfrei gekühlter Kondensator zwischengeschaltet ist, um das durch die Saugleitung der Fördereinrichtung strömende Fördermedium in den flüssigen Aggregatzustand zu überführen (vgl. DE 44 45 054 C2).

In der DE 44 45 054 C2 ist auch beschrieben, wie die Bearbeitungskammer der vorbekannten Bearbeitungsvorrichtung bei Programmstart durch Öffnen eines Magnetventils und Einschalten der Saugpumpe auf einen vorbestimmten Druck evakuiert werden kann. Anschließend wird das Magnetventil geschlossen und aus einem Dampferzeuger Dampf in den Druckkessel eingeleitet, bis ein vorgegebener Überdruck erreicht wird. Durch erneutes Öffnen des Magnetventils und Einschalten der Membranpumpe wird das Dampf-/Luftgemisch wiederum bis zu einem definierten Unterdruck abgesaugt, wobei der aus der Bearbeitungskammer kommende Wasserdampf in einer als Kondensator ausgebildeten Kühleinrichtung kondensiert und in einem Kondensatorsammelgefäß gesammelt wird, damit die Membranpumpe der vorbekannten Bearbeitungsvorrichtung nur Luft fördert.

Bei der Kopplung einer herkömmlichen Membran-Vakuumpumpe mit einem herkömmlichen Autoklaven tritt allerdings folgendes Problem auf: Nach Beendigung eines Sterilisiervorganges wird der Dampf über eine Verbindungsleitung durch die Vakuumpumpe ausgestoßen. In der kalten Verbindungsleitung und der kalten Vakuumpumpe kondensiert mindestens ein Teil des Dampfes. Dieses Kondensat wird von der Vakuumpumpe abgepumpt. Problematisch ist hierbei, daß während des Betriebs im Bereich des sich erwärmenden Pumpenkopfes eine Rückverdampfung des Kondensats auftreten kann, so daß dann ein entsprechend größeres Volumen abgepumpt werden muß. Dies erfordert eine erhebliche Zeit.

Eine solche Rückverdampfung wird auch dadurch nicht ausgeschlossen, daß in der zwischen Bearbeitungskammer und Saugpumpe vorgesehenen Saugleitung ein Kondensator zwischengeschaltet ist.

Die durch eine solche Rückverdampfung verursachten Probleme stellen sich über die eingangs beschriebenen Autoklaven hinaus auch bei allen anderen, beispielsweise zur Geltrocknung verwendeten Bearbeitungsvorrichtungen, aus deren Bearbeitungskammer ein feuchtes oder flüssiges Fördermedium zu evakuieren ist. Dabei soll hier unter einem feuchten Fördermedium beispielsweise jedes Gas oder Gasgemisch verstanden sein, welches zumindest eine Teilmenge eines in gas- oder dampfförmigem bzw. tropfbar-flüssigem Zustand befindlichen Stoffes transportiert.

Es besteht daher die Aufgabe, ein Verfahren sowie eine Bearbeitungsvorrichtung der eingangs erwähnten Art zu schaffen, die ein rasches und wirksames Abpumpen solch feuchter Fördermedien erlauben.

Bei dem Verfahren der eingangs erwähnten Art besteht die erfindungsgemäße Lösung insbesondere darin, daß die Saugpumpe als ein- oder mehrstufige Membranpumpe ausgebildet ist und daß die Kühlung des Fördermediums zumindest im Bereich wenigstens eines Pumpenkopfes der Membranpumpe und/oder eines, zwei in Strömungsrichtung nachfolgende Pumpstufen einer mehrstufigen Membranpumpe miteinander verbindenden Verbindungskanales erfolgt und daß der wenigstens eine Pumpenkopf und/oder der Verbindungskanal der Membranpumpe unter die bei gegebenem Evakuierungsdruck vorhandene Verdampfungs- oder Siedetemperatur abgekühlt wird.

Bei der Bearbeitungsvorrichtung der eingangs erwähnten Art besteht die erfindungsgemäße Lösung insbesondere darin, daß die Saugpumpe als ein oder mehrstufige Membranpumpe ausgebildet ist und daß wenigstens eine Kühleinrichtung zum Kühlen zumindest eines Pumpenkopfes der Membranpumpe und/oder eines, zwei in Strömungsrichtung nachfolgende Pumpstufen einer mehrstufigen Membranpumpe miteinander verbindenden Verbindungskanals vorgesehen ist und daß der Pumpenkopf und/oder Verbindungskanal der Membranpumpe mittels dieser Kühleinrichtung derart kühlbar ist, daß die Kopftemperatur der Membranpumpe und/oder die Innentemperatur des Verbindungskanals unter die bei gegebenem Evakuierungsdruck vorhandene Verdampfungs- oder Siedetemperatur abgekühlt wird.

Bei den oben geschriebenen Erfindungsgegenständen zieht die Membranpumpe das feuchte und beispielsweise dampfhaltige Fördermedium aus der Bearbeitungskammer, wobei der mitgeführte Dampf beim Ausschieben in der Membranpumpe auf Atmosphärendruck verdichtet wird und bei Kopftemperaturen unter 100°C in der Pumpe kondenisert. Dieses Kondensat wird über die Membranpumpe ins Freie befördert. In den Toträumen oder in der zwei benachbarte Pumpstufen miteinander verbindenden Verbindungsleitung kann jedoch noch ein Rest des Kondensats verbleiben. Beim Ansaughub wird der Druck im Arbeitsraum der Membranpumpe reduziert. Zumindest ein Teil des in der Membranpumpe verbliebenen Kondensats könnte nun verdampfen, wenn der Ansaugdruck in der Vakuumpumpe den zur augenblicklichen Kopftemperatur am Pumpenkopf entsprechenden Verdampfungsdruck unterschreiten würde. Eine solche Rückverdampfung des im Pumpenkopf befindlichen Kondensats oder Kondensatfilms würde jedoch erneut zu einer Vergrößerung des abzupumpenden Fördervolumens und somit zur Reduzierung des Ansaugvolumens sowie der Saugleistung der Vakuumpumpe führen. Um eine solche Rückverdampfung auszuschließen, ist bei dem erfindungsgemäßen Verfahren daher vorgesehen, daß die Kühlung des Fördermediums zumindest im Bereich wenigstens eines Pumpenkopfes und/oder eines, benachbarte Pumpenstufen mit einander verbindenden Verbindungskanals der ein- oder mehrstufigen Membranpumpe erfolgt. Dabei wird der wenigstens eine Pumpenkopf und/oder der Verbindungskanal der Membranpumpe unter die bei gegebenem Evakuierungsdruck vorhandene Verdampfungs- oder Siedetemperatur abgekühlt. Da somit das durch den wenigstens einen Pumpenkopf und/oder den Verbindungskanal geförderte Kondensat niemals die dem in der Bearbeitungskammer angestrebten Verdampfungsdruck entsprechende Siedetemperatur erreichen kann, wird eine Rückverdampfung des im Pumpenkopf oder in dem Verbindungskanal befindlichen Kondensats oder Kondensatfilms mit Sicherheit ausgeschlossen.

Zur Kühlung des zumindest einen Pumpenkopfes und/oder des zwischen den Pumpstufen einer mehrstufigen Membranpumpe vorgesehenen Verbindungskanales können Peltierelemente oder jede andere geeignete Kühieinrichtung verwendet werden. Eine besonders einfache und vorteilhafte Ausführungsform gemäß der Erfindung sieht jedoch vor, daß die Kühleinrichtung als Luftkühlung, vorzugsweise als Gebläse-Kühlung ausgebildet ist.

Dabei ist es besonders vorteilhaft, wenn der im Kühlstrom einer Luftkühlung angeordnete Pumpenkopf und/oder der Verbindungskanal der Membranpumpe außenseitig Kühlrippen zur Vergrößerung der Kühlfläche und gegebenenfalls zur Strömungsführung des Kühlstromes aufweist beziehungsweise aufweisen. Bei dieser Ausführungsform kann das Fördermedium im Bereich des Pumpenkopfes und/oder des Verbindungskanals kondensieren oder in flüssigem Aggregatzustand gehalten werden, ohne daß ein größerer Herstellungs- und Konstruktionsaufwand notwendig wäre. Dabei bewirkt die Kühlung des Pumpenkopfes beziehungsweise des Verbindungskanals gleichzeitig auch einen kühlen Lauf der Pumpe, was eine lange Lebensdauer der Pumpe und lange Service-Intervalle begünstigt.

Bekanntlich weist etwa Wasser in flüssigem Zustand ein wesentlich geringeres Volumen auf als im dampfförmigen Zustand. Wird in dem zwischen Bearbeitungskammer und Fördereinrichtung vorgesehenen geschlossenen System der während eines Bearbeitungsvorganges erzeugte Wasserdampf im Bereich der Fördereinrichtung mittels einer Kühleinrichtung abgekühlt und kondensiert, so wird gleichzeitig auch das Volumen des ursprünglich dampfförmigen Fördermediums während der Kondensation auf einen Bruchteil des Volumens reduziert. Durch diese Volumenreduktion im Bereich der Fördereinrichtung entsteht ein Kondensationspumpeffekt, welcher die Förderleistung der Fördereinrichtung bestimmt oder zumindest unterstützt. Um diesen Kondensationspumpeffekt möglichst vollständig zur Evakuierung der Bearbeitungskammer nutzen zu können, ist es vorteilhaft, wenn in die zwischen Bearbeitungskammer und dem Auslaß der Fördereinrichtung vorgesehene Strömungsführung ein Rückschlagventil zwischengeschaltet ist.

Dabei wird eine Ausführungsform bevorzugt, bei der das Rückschlagventil vorzugsweise das Auslaßventil der Membranpumpe beziehungsweise einer ihrer im Bereich des Pumpenkopfes gekühlten Pumpstufen ist. Eine solche Membranpumpe kann nicht nur klein, kompakt und kondensat-unempfindlich ausgebildet werden, sondern weist bauartbedingt bereits das erforderliche Rückschlagventil am Pumpenauslaß auf.

Eine bevorzugte Ausführungsform gemäß der Erfindung sieht vor, daß die Bearbeitungsvorrichtung als Dampfsterilisationsvorrichtung und ihre Bearbeitungskammer als Sterilisationskammer ausgebildet sind.

Mit Hilfe des eingangs beschriebenen Erfindungsgegenstandes wird eine Rückverdampfung des in dem zumindest einen Pumpenkopf befindlichen Kondensats oder Kondensatfilms vermieden. Um nun dort das in den Ventilräumen bzw. im Verdichtungsraum vorhandene flüssige Fördermedium abführen zu können, muß die Pumpe so gestaltet sein, daß sie sich beispielsweise schwerkraftbedingt und/oder aufgrund ihrer Pumpwirkung selbständig entleeren kann. Bei Verwendung einer Pumpe, die zumindest ein Ventil mit einer vom Fördermedium gesteuerten Ventilscheibe hat, können jedoch bereits geringe Flüssigkeitsmengen von beispielsweise ein oder zwei Tropfen dazu führen, daß die Ventilscheibe am Ventilsitz "festklebt" und daß die bei niedrigen absoluten Drücken entsprechend geringen Differenzdrücke nicht ausreichen, um die Ventilscheibe zu bewegen. Damit derartige Klebeeffekte vermieden werden, ist nach einem weiteren Vorschlag gemäß der Erfindung vorgesehen, daß die dichtenden Berührungsstellen zwischen der Dichtseite der Ventilscheibe und der die Ventilöffnung umgrenzenden Dichtfläche des Pumpenkopfes im wesentlichen als linienförmige Berührungsstellen ausgebildet sind. Durch diese linienförmigen Berührungsstellen wird eine flächige Auflage der Ventilscheibe vermieden, die andernfalls zu unerwünschten Klebeeffekten und - bei geringen Differenzdrücken - zu entsprechenden Störungen am Ventil führen könnten.

Um solche Klebeeffekte erforderlichenfalls auch auf der der Dichtseite der Ventilscheibe abgewandten Seite zu verhindern, ist es vorteilhaft, wenn die Berührungsstellen zwischen der der Dichtseite abgewandten Rückseite der Ventilscheibe einerseits und einer Auflage für die in Offenstellung befindliche Ventilscheibe andererseits im wesentlichen als punktförmige und/oder ebenfalls als linienförmige Berührungsstellen ausgebildet sind.

Zweckmäßig ist es, wenn die Dichtfläche im Pumpenkopf und/oder die Dichtseite der Ventilscheibe wenigstens einen, zumindest eine Ventilöffnung umgrenzenden Ringvorsprung mit vorzugsweise konisch sich zur Berührungsstelle hin verjüngendem Querschnitt aufweist. Dabei wird eine Ausführungsform bevorzugt, die an der Dichtfläche des Pumpenkopfes zumindest einen konischen Ringvorsprung hat.

Um auch auf der der Dichtseite abgewandten Rückseite der Ventilscheibe derartige Klebeeffekte zu vermeiden, ist es zweckmäßig, wenn die rückseitige Auflage für die in Offenstellung befindliche Ventilscheibe und/oder die Ventilscheibenrückseite eine Profilierung als linienförmige und/oder punktförmige Berührungsstellen zur Verkleinerung der Anlageoberfläche aufweist.

Dabei kann die rückseitige Auflage für die Ventilscheibe durch die Kanten eines treppenförmigen Vorsprunges oder mehrerer treppenförmiger Vorsprünge mit vorzugsweise linienförmigen Berührungsstellen gebildet sein.

Vorteilhaft ist es, wenn die Membranpumpe in ihren vom Fördermedium beaufschlagten Bereichen aus korrosionsbeständigen Werkstoffen hergestellt ist. Dadurch werden eine lange Lebensdauer der Pumpe sowie lange Service-Intervalle noch zusätzlich begünstigt.

Weitere Merkmale der Erfindung ergeben sich aus der folgenden Beschreibung der erfindungsgemäßen Ausführungsbeispiele in Verbindung mit den Ansprüchen sowie der Zeichnung. Die einzelnen Merkmale können je für sich oder zu mehreren bei einer Ausführungsform gemäß der Erfindung verwirklicht sein.

Es zeigt:
- Fig. 1: eine schematisch dargestellte und hier als Dampfsterilisationsvorrichtung ausgebildete Bearbeitungsvorrichtung mit einer Bearbeitungs- oder Sterilisationskammer, welche an eine Fördereinrichtung angeschlossen ist, die eine Vakuumpumpe zum Evakuieren der Bearbeitungs- oder Sterilisationskammer aufweist,
- Fig. 2: die Dampfdruck-Kurve von Wasser, die die Abhängigkeit der Siedetemperatur (Abszisse) und des Dampfdruckes (Ordinate) voreinander wiedergibt,
- Fig. 3: die Membranpumpe der Bearbeitungsvorrichtung gemäß Fig. 1 in einem Teil-Querschnitt im Bereich des Einlaßventiles, wobei das Einlaßventil in seiner Offenstellung dargestellt ist,
- Fig. 4: ein mit Fig. 3 vergleichbares Einlaßventil für eine Bearbeitungsvorrichtung gemäß Fig. 1, und
- Fig. 5: eine zweistufige Membranpumpe in einer schematischen Darstellung, bei der sowohl die beiden Pumpstufen im Bereich ihrer Pumpköpfe als auch der diese benachbarten Pumpstufen miteinander verbindende Verbindungskanal gekühlt werden.

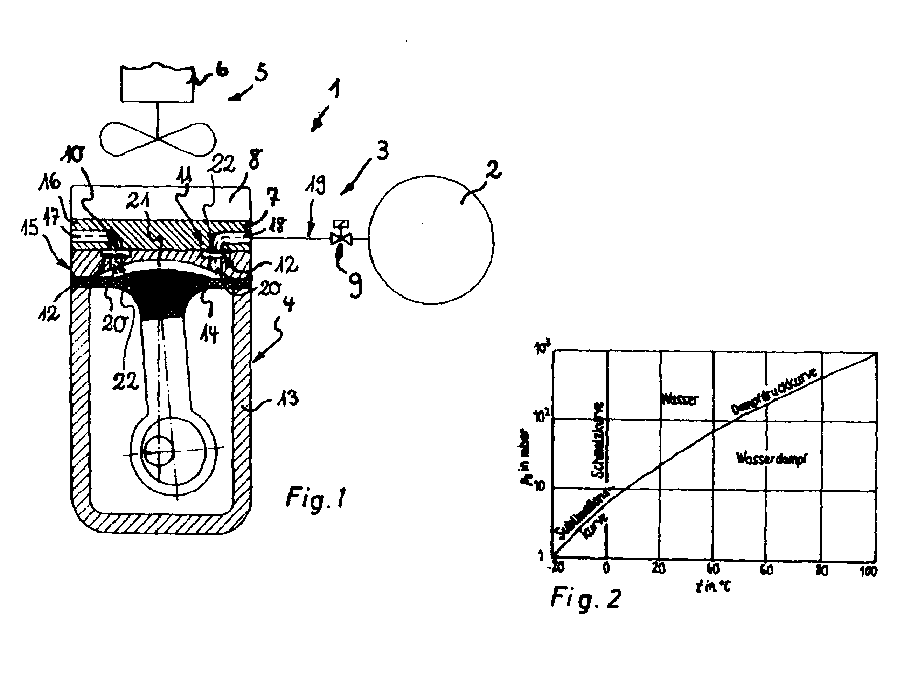

In Fig. 1 ist eine als Dampfsterilisationsvorrichtung 1 ausgebildete Bearbeitungsvorrichtung dargestellt, die eine luftdichtverschließbare Bearbeitungs- oder Sterilisationskammer 2 aufweist. An die Sterilisationskammer 2 ist eine Fördereinrichtung 3 angeschlossen, die zum Evakuieren der Sterilisationskammer 2 dient. Diese Fördereinrichtung 3 weist eine Membranpumpe 4 auf, die sowohl Kondensat fördern wie auch Vakuum erzeugen kann.

Die Fördereinrichtung 3 ist zumindest in einem Teilbereich mittels einer Kühleinrichtung 5 derart kühlbar, daß sich das Fördermedium der Fördereinrichtung 3 in flüssigem Aggregatzustand befindet oder überführt wird.

Wie Figur 1 zeigt, weist die Kühleinrichtung 5 ein Kühlgebläse 6 auf. Der im Kühlstrom des Kühlgebläses 6 angeordnete Pumpenkopf 7 der Saugpumpe 4 weist außenseitig zur Kühlflächen-Vergrößerung sowie zur Strömungsführung des Kühlstromes Kühlrippen 8 auf.

Nachdem die zu sterilisierenden Instrumente und sonstigen medizinischen Gebrauchsartikel in die luftdicht verschließbare Sterilisationskammer 2 eingebracht wurden, wird die Sterilisationskammer 2 beispielsweise auf 200 Millibar evakuiert. Dabei kann erforderlichenfalls durch wiederholtes Evakuieren der in der Sterilisationskammer 2 befindlichen Luft im Wechsel mit dem periodischen Einströmen von Dampf eine besonders gute Luftentfernung auch aus englumigen Instrumenten und dergleichen erreicht werden.

Für den nachfolgenden Sterilisiervorgang wird das in die Strömungsführung zwischen Sterilisationskammer 2 und Vakuumpumpe 4 befindliche, vorzugsweise elektrisch betätigbare Schließventil 9 geschlossen, um die Sterilisationskammer 2 aufzuheizen und unter Überdruck mit Dampf zu füllen.

Nach Beendigung dieses Zyklus wird in der Sterilisationskammer 2 ein Nachvakuum erzeugt, damit das eventuell auf den Instrumenten und dergleichen kondensierte Wasser abdampfen und das Sterilisiergut völlig trocken werden kann. Dazu wird das Schließventil 9 zunächst geöffnet und der Dampf strömt, solange in der Sterilisationskammer 2 noch Überdruck herrscht, über die Saugpumpe 4 aus. Dabei kondensiert mindestens ein Teil des Dampfes in der Saugpumpe 4 und wird in flüssiger Form ins Freie befördert.

Wenn der Überdruck in der Sterilisationskammer 2 abgebaut ist, schließt das auch als Rückschlagventil dienende Auslaßventil 10 der Membranpumpe im Rhythmus des Pumpvorganges. Die Saugpumpe 4 zieht nun das dampfhaltige Fördermedium aus der Sterilisationskammer 2, wobei der mitgeführte Dampf beim Ausschieben in der Saugpumpe 4 auf Atmosphärendruck verdichtet wird und bei Kopftemperatur unter 100°C in der Pumpe kondensiert. Das Kondensat wird über die Saugpumpe 4 ins Freie befördert. In den Toträumen kann jedoch noch ein Rest des Kondensats verbleiben.

Beim Ansaughub wird der Druck im Arbeitsraum der Membranpumpe 4 reduziert. Zumindest ein Teil des in der Saugpumpe 4 verbliebenen Kondensats könnte nun verdampfen, wenn der Ansaugdruck in der Vakuumpumpe 4 den zur augenblicklichen Kopftemperatur am Pumpenkopf 7 entsprechenden Verdampfungsdruck gemäß Figur 2 unterschreiten würde. Eine solche Rückverdampfung des im Pumpenkopf 7 befindlichen Kondensats oder Kondensatfilms würde jedoch erneut zu einer Vergrößerung des abzupumpenden Fördervolumens und somit zur Reduzierung des Ansaugvolumens sowie der Saugleistung der Vakuumpumpe 4 führen.

Um eine solche Rückverdampfung auszuschließen, ist der Pumpenkopf 7 der Vakuumpumpe mittels der Kühleinrichtung 5 derart kühlbar, daß die Kopftemperatur etwa in oder vorzugsweise unter der Verdampfungs- oder Siedetemperatur liegt, die dem bei diesen Sterilisationsvorgang gewünschten Verdampfungsdruck in der Sterilisationskammer 2 entspricht.

Soll beispielsweise während des Nachvakuums in der Sterilisationskammer 2 ein Verdampfungsdruck von 200 Millibar erreicht werden, so läßt sich aus der üblichen Wasserdampf-Tabelle oder aus der in Figur 2 abgebildeten Dampfdruck-Kurve von Wasser entnehmen, daß einem solchen Verdampfungsdruck eine Verdampfungsund Siedetemperatur von 60,09°C entspricht. Um eine Rückverdampfung des im Pumpenkopf 7 befindlichen Kondensats oder Kondensatfilms mit Sicherheit auszuschließen, ist die Kühleinrichtung 5 beispielsweise durch ein entsprechend leistungsfähiges Kühlgebläse 6 und/oder durch eine entsprechende Ausgestaltung der am Pumpenkopf 7 vorgesehenen Kühlrippen 8, so ausgelegt, daß der Pumpenkopf 7 während dieses Sterilisiervorganges auf unter 60,09°C gekühlt wird. Da somit das durch den Pumpenkopf 7 geförderte Kondensat niemals die dem in der Sterilisationskammer 2 angestrebten Verdampfungsdruck entsprechende Siedetemperatur erreichen kann, wird eine Rückverdampfung des im Pumpenkopf 7 befindlichen Kondensats oder Kondensatfilms mit Sicherheit ausgeschlossen.

In der hier dargestellten Dampfsterilisationsvorrichtung 1 kann die Sterilisationskammer 2 rasch und wirkungsvoll evakuiert werden.

Da das dampfhaltige Fördermedium im Bereich der Fördereinrichtung 3 mittels der Kühleinrichtung 5 gekühlt wird und der Wasserdampf somit kondensieren kann, und da das Volumen des Kondensats einen Bruchteil des ursprünglichen Wasserdampf-Volumens beträgt, wird das abzupumpende Fördervolumen deutlich reduziert. Gleichzeitig entsteht in dem geschlossenen System zwischen der Sterilisationskammer 2 und dem Auslaßventil 10 der Membranpumpe 4 ein Kondensationspumpeffekt, welcher die Förderleistung der Fördereinrichtung 3 zusätzlich unterstützt.

Da die Kühleinrichtung 5 so ausgelegt ist, daß das im Pumpenkopf 7 befindliche Kondensat nie die dem in der Sterilisationskammer 2 angestrebten Verdampfungsdruck entsprechende Siedetemperatur erreicht, wird eine unerwünschte und leistungsmindernde Rückverdampfung des im Pumpenkopf 7 befindlichen Kondensats oder Kondensatfilms mit Sicherheit vermieden. Gleichzeitig wird durch die Kühlung der Vakuumpumpe 4 im Bereich des Pumpenkopfes 7 ein kühler Lauf der Pumpe bewirkt, der eine lange Lebensdauer der Vakuumpumpe 4 und lange Service-Intervalle begünstigt. Die Kühleinrichtung 5 kann beispielsweise in Form einer Luftkühlung als ungeregeltes Kühlsystem betrieben werden. Da bei einer solchen Luftkühlung nur das Kühlgebläse 6 zu betreiben ist, kann der Energieverbrauch der Kühleinrichtung 5 gering gehalten werden. Dabei kann das in der Dampfsterilisationsvorrichtung 1 anfallende Kondensat wiederverwendet werden.

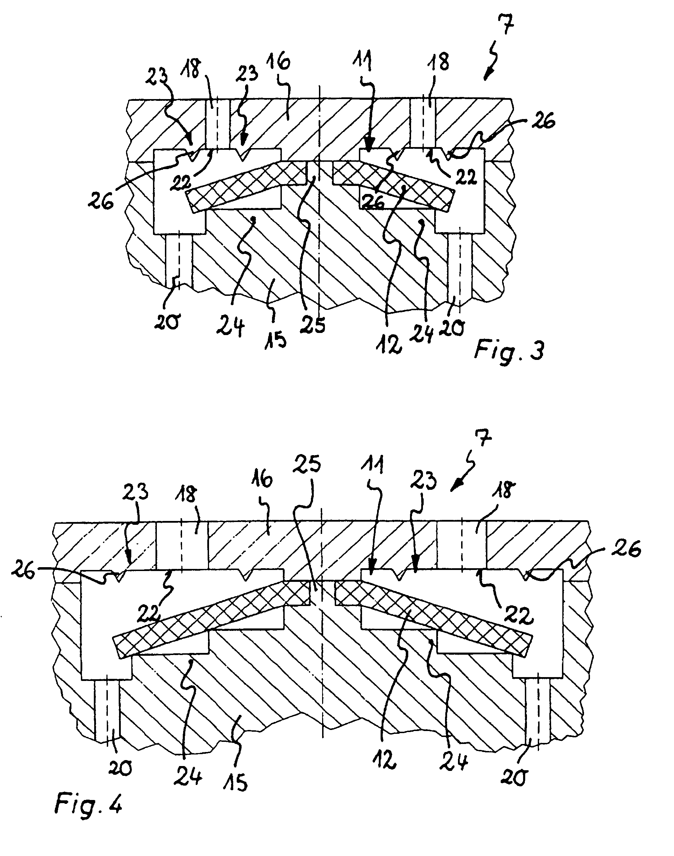

Wie bereits erwähnt, ist die Saugpumpe 4 der hier dargestellten Bearbeitungsvorrichtung als Membranpumpe ausgebildet. Die Membranpumpe 4 hat Auslaß- und Einlaßventile 10, 11, welche jeweils eine vom Fördermedium gesteuerte Ventilscheibe 12 aufweisen. Das Einlaßventil 11 der Saugpumpe 4 ist in den Fig 3 und 4 in zwei ähnlichen Ausführungen noch näher dargestellt.

Wie aus einem Vergleich der Fig. 1 und 3 bis 4 deutlich wird, hat die Membranpumpe 4 ein Gehäuse 13, eine Membrane 14, einen Zwischendeckel 15 und einen sich daran anschließenden Abschlußdeckel 16. Die beiden Deckel 15, 16 bilden gemeinsam dem Pumpenkopf 7. Im Abschlußdeckel 16 befindet sich ein Auslaßkanal 17 sowie ein über die Saugleitung 19 mit der Bearbeitungs- und Sterilisationskammer 2 verbundener Einlaßkanal 18. Die Ventile 10, 11 stehen über Zuleitungen 20 mit dem kalottenförmigen Pumpraum 21 im Zwischendeckel 15 in Verbindung.

In Schließstellung der Ventile 10, 11 liegt deren Ventilscheibe 12 dichtend an der die Ventilöffnung 22 umgrenzenden Ventildichtfläche 23 des Pumpenkopfes an. Um die Ventilöffnungsbewegung zu begrenzen, liegt die Ventilscheibe in der in Fig. 3 und 4 gezeigten Offenstellung an einer als Anschlagfläche dienenden rückseitigen Auflage 24 an. Dabei sind die kreisscheibenförmigen Ventilscheiben 12 mittels eines Zapfens 25 zwischen dem Abschlußdeckel 16 und dem Zwischendeckel 15 zentral gehalten.

Die Eigenelastizität des Ventilscheiben-Werkstoffes sorgt üblicherweise für ein genügend schnelles Zurückgehen der Ventilscheibe 12 in ihre Schließstellung bei entsprechenden Druckdifferenzen des Fördermediums im Pumpbetrieb. Da mit Hilfe der auf den Pumpenkopf einwirkenden Kühleinrichtung 5 eine Rückverdampfung des im Pumpraum 21 oder den Ventilräumen befindlichen Kondensats oder Kondensatfilms vermieden wird, besteht bei Verwendung üblicher Saugpumpen die Gefahr, daß die Ventilscheiben 12 bereits bei geringen Flüssigkeitsmengen von ein oder zwei Tropfen an der Dichtfläche des Pumpenkopfes verklebt und daß die bei geringen absoluten Drücken vorhandenen kleinen Differenzdrücke nicht ausreichen, um die Adhäsionskräfte zu überwinden und die Ventilscheibe 12 zu bewegen.

Um ein einwandfreies Arbeiten der im Einlaß- und/oder im Auslaßventil 11, 10 vorgesehenen Ventilscheiben 12 zu gewährleisten, sind die dichtenden Berührungslinien zwischen der Dichtseite der Ventilscheibe 12 und der die zumindest eine Ventilöffnung 22 umgrenzenden Ventil-Dichtfläche 23 des Pumpenkopfes 7 im wesentlichen als linienförmige Berührungsstellen ausgebildet. Das in Fig. 3 und 4 dargestellte Ventil 11 weist dazu zwei Ringvorsprünge 26 auf, die am Pumpenkopf 7 jeweils eine Ventilöffnung 22 umgrenzen. Dabei haben diese Ringvorsprünge 26 einen sich zur Berührungsstelle hin konisch verjüngenden Querschnitt.

Aus dem gleichen Grund sind die Berührungsstellen zwischen der Rückseite der Ventilscheibe 12 und der Auflage 24 für die in Offenstellung befindliche Ventilscheibe 12 hier im wesentlichen als linienförmige Berührungsstellen ausgebildet. Die rückseitige Auflage 24 der Ventile 10, 11 wird dazu durch die Kanten eines treppenförmigen Vorsprunges (vgl. Fig. 3) oder mehrerer treppenförmiger Vorsprünge (vgl. Fig. 4) mit vorzugsweise linienförmigen Berührungsstellen gebildet. Der Ventilscheibenrückseite können aber auch - hier nicht weiter dargestellte - punktförmige Vorsprünge als rückseitige Auflagen dienen.

Da die Ventilscheibe 12 der Ventile 10, 11 sowohl die Ventil-Dichtfläche 23 als auch die rückseitige Auflage 24 nur punkt- bzw. linienförmig berührt, können die infolge eines Kondensatfilms in den Ventilräumen auf die Ventilscheibe 12 einwirkenden Adhäsionskräfte so gering gehalten werden, daß die Ventilscheibe 12 auch bei geringen Differenzdrücken noch störungsfrei zwischen ihrer Offenstellung und der Schließstellung oszilliert. Zwar ist in den Fig. 3 und 4 nur jeweils das Einlaßventil 11 dargestellt, jedoch ist auch das Auslaßventil 10 der Bearbeitungs- oder Dampfsterilisationsvorrichtung 1 entsprechend ausgestaltet.

Die hier dargestellte Bearbeitungsvorrichtung erlaubt ein rasches und wirksames Abpumpen auch feuchter Fördermedien, wobei sich der Anwendungsbereich dieser Bearbeitungsvorrichtung nicht allein auf Dampfsterilisationsvorrichtungen beschränkt.

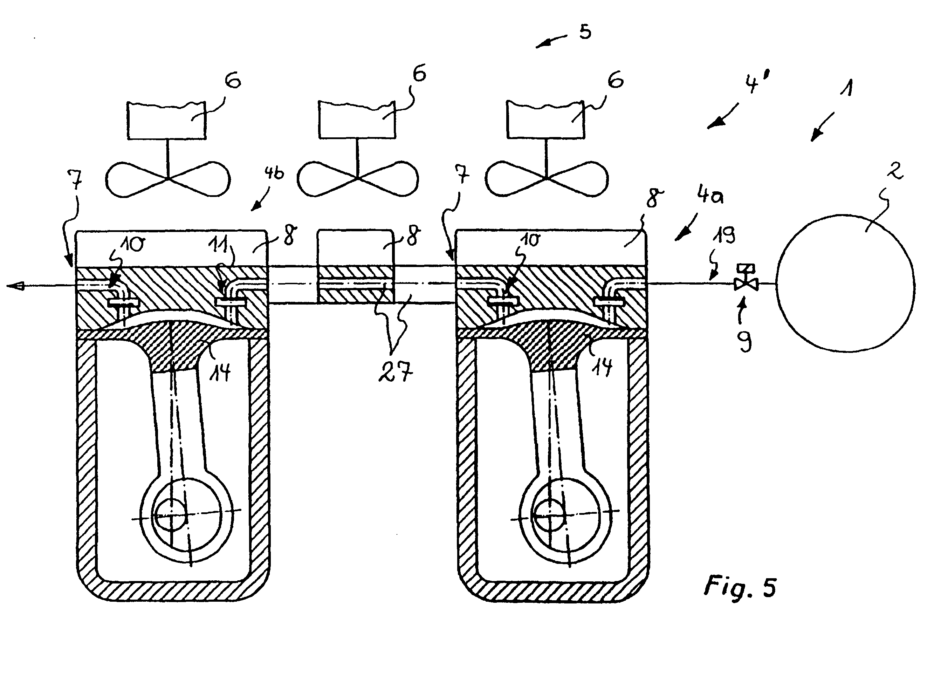

In Figur 5 ist eine Bearbeitungsvorrichtung 1 dargestellt, die - ähnlich wie die Vorrichtung gemäß Figur 1 - ebenfalls als Dampfsterilisator ausgebildet ist. Die Bearbeitungsvorrichtung 1 gemäß Figur 5 weist eine mehrstufige Saugpumpe 4' auf, deren Pumpstufen 4a, 4b durch zwei, einander in Strömungsrichtung nachfolgende Membranpumpen gebildet sind. Der Auslaß der die Pumpstufe 4a bildenden Membranpumpe ist über einen Verbindungskanal 27 mit dem Einlaß der in Strömungsrichtung nachfolgenden und als Pumpstufe 4b vorgesehenen Membranpumpe verbunden. Um eine Rückverdampfung des spätestens im Bereich der Saugpumpe 4' kondensierenden Fördermediums auszuschließen, ist eine Kühleinrichtung 5 vorgesehen, die drei, jeweils einer Pumpstufe 4a, 4b beziehungsweise dem Verbindungskanal 27 zugeordnete Kühlgebläse 6 hat. An den Pumpenköpfen 7 der Pumpstufen 4a, 4b sowie am Verbindungskanal 27 sind außenseitig Kühlrippen 8 vorgesehen, welche die Kühlfläche vergrößern und zur Strömungsführung des Kühlstromes dienen sollen.

Dabei werden sowohl die Pumpenköpfe 7 der Pumpstufen 4a, 4b als auch der sie verbindende Verbindungskanal 27 derart gekühlt, daß sowohl die an den Pumpenköpfen 7 gemessene Kopftemperatur der Pumpstufen 4a, 4b als auch die Innentemperatur im Verbindungskanal 27 unter die bei gegebenem Evakuierungsdruck vorhandene Verdampfungs- oder Siedetemperatur abgekühlt wird.

Bei einer mehrstufigen Saugpumpe ist es auch möglich, nur zumindest einen der Pumpenköpfe 7 oder nur den Verbindungskanal 27 zu kühlen. Bevorzugt wird jedoch die hier dargestellte Ausführungsform, bei der die Kühleinrichtung 5 jeweils ein den Pumpenköpfen 7 sowie dem Verbindungskanal 27 zugeordnetes Kühlgebläse 6 hat. Dabei ist es vorteilhaft, wenn zumindest eine, vorzugsweise jede der Pumpstufen an ihrem Auslaß und/oder an ihrem Einlaß ein Ventil 10, 11 gemäß den Figuren 3, 4 aufweist.

## Patentansprüche

1. Verfahren zum Evakuieren eines feuchten oder flüssigen Fördermediums aus der Bearbeitungskammer (2) einer Bearbeitungsvorrichtung (1) mittels einer Fördereinrichtung (3), die eine ein- oder mehrstufige Saugpumpe (4, 4') hat, wobei das Fördermedium während der Evakuierung im Verlauf des Strömungsweges derart gekühlt wird, daß sich das Fördermedium in der Fördereinrichtung (3) in flüssigem Aggregatzustand befindet oder überführt wird, **dadurch gekennzeichnet, daß** die Saugpumpe (4, 4') als ein- oder mehrstufige Membranpumpe ausgebildet ist und daß die Kühlung des Fördermediums zumindest im Bereich wenigstens eines Pumpenkopfes (7) der Membranpumpe (4, 4') und/oder eines, zwei in Strömungsrichtung nachfolgende Pumpstufen (4a, 4b) einer mehrstufigen Membranpumpe (4') miteinander verbindenden Verbindungskanals (27) erfolgt und daß der wenigstens eine Pumpenkopf (7) und/oder der Verbindungskanal der Membranpumpe (4, 4') unter die bei gegebenem Evakuierungsdruck vorhandene Verdampfungs- oder Siedetemperatur abgekühlt wird.

2. Bearbeitungsvorrichtung zur Durchführung des Verfahrens nach Anspruch 1, mit einer Bearbeitungskammer (2), welche zur Evakuierung an eine Fördereinrichtung (3) angeschlossen ist, sowie mit einer ein- oder mehrstufigen Saugpumpe (4, 4'), die der Fördereinrichtung (3) zugeordnet ist, wobei die Fördereinrichtung (3) zumindest in einem Teilbereich mittels wenigstens einer Kühleinrichtung (5) derart kühlbar ist, daß sich das Fördermedium in der Fördereinrichtung (3) in flüssigem Aggregatzustand befindet oder überführt wird, **dadurch gekennzeichnet, daß** die Saugpumpe (4, 4') als ein- oder mehrstufige Membranpumpe ausgebildet ist und daß wenigstens eine Kühleinrichtung zum Kühlen zumindest eines Pumpenkopfes (7) der Membranpumpe (4, 4') und/oder eines, zwei in Strömungsrichtung nachfolgende Pumpstufen (4a, 4b) einer mehrstufigen Membranpumpe (4') miteinander verbindenden Verbindungskanals (27) vorgesehen ist, und daß der Pumpenkopf (7) und/oder der Verbindungskanal (27) der Membranpumpe (4, 4') mittels dieser Kühleinrichtung (5) derart kühlbar ist, daß die Kopftemperatur der Membranpumpe (4, 4') und/oder die Innentemperatur des Verbindungskanals (27) unter die bei gegebenem Evakuierungsdruck vorhandene Verdampfungs- oder Siedetemperatur abgekühlt wird.

3. Bearbeitungsvorrichtung nach Anspruch 2, **dadurch gekennzeichnet, daß** die Kühleinrichtung (5) als Luftkühlung, vorzugsweise als Gebläsekühlung, ausgebildet ist.

4. Bearbeitungsvorrichtung nach Anspruch 2 oder 3, **dadurch gekennzeichnet, daß** der zumindest eine im Kühlstrom einer Luftkühlung angeordnete Pumpenkopf (7) und/oder der Verbindungskanal (27) der Membranpumpe (4, 4') außenseitig Kühlrippen (8) zur Vergrößerung der Kühlfläche und gegebenenfalls zur Strömungsführung des Kühlstromes aufweist beziehungsweise aufweisen.

5. Bearbeitungsvorrichtung nach einem der Ansprüche 2 bis 4, **dadurch gekennzeichnet, daß** in die zwischen Bearbeitungskammer (2) und dem Auslaß der Fördereinrichtung (3) vorgesehene Strömungsführung ein Rückschlagventil zwischengeschaltet ist.

6. Bearbeitungsvorrichtung nach einem der Ansprüche 2 bis 5, **dadurch gekennzeichnet, daß** das Rückschlagventil vorzugsweise das Auslaßventil (11) der Membranpumpe (4, 4') beziehungsweise das Auslaßventil einer ihrer im Bereich des Pumpenkopfes gekühlten Pumpstufen (4a, 4b) ist.

7. Bearbeitungsvorrichtung nach einem der Ansprüche 2 bis 6, **dadurch gekennzeichnet, daß** die Bearbeitungsvorrichtung als Dampfsterilisationsvorrichtung (1) und deren Bearbeitungskammer als Sterilisationskammer (2) ausgebildet ist.

8. Bearbeitungsvorrichtung gemäß einem der Ansprüche 2 bis 7, mit ein- oder mehrstufiger Membranpumpe mit wenigstens einem, in einem Pumpenkopf befindlichen Einlaß- und zumindest einem Auslaßventil, von denen mindestens ein Ventil eine vom Fördermedium gesteuerte Ventilscheibe (12) aufweist, die in Schließstellung des Ventils (10, 11) an einer, zumindest eine Ventilöffnung (22) umgrenzenden Ventil-Dichtfläche (23) des Pumpenkopfes (7) dichtend anliegt, **dadurch gekennzeichnet, daß** die dichtenden Berührungsstellen zwischen der Dichtseite der Ventilscheibe (12) und der die Ventilöffnung (22) umgrenzenden Ventil-Dichtfläche (23) des Pumpenkopfes (7) im wesentlichen als linienförmige Berührungsstellen ausgebildet sind.

9. Bearbeitungsvorrichtung mit Pumpe nach Anspruch 8, **dadurch gekennzeichnet, daß** die Berührungsstellen zwischen der Rückseite der Ventilscheibe (12) und einer Auflage (24) für die in Offenstellung befindliche Ventilscheibe (12) im wesentlichen als linienförmige und/oder punktförmige Berührungsstellen ausgebildet sind.

10. Bearbeitungsvorrichtung mit Pumpe nach Anspruch 8 und 9, **dadurch gekennzeichnet, daß** die Ventil-Dichtfläche (23) und/oder die Dichtseite der Ventilscheibe (12) wenigstens einen, zumindest eine Ventilöffnung (22) umgrenzenden Ringvorsprung (26) mit vorzugsweise konisch sich zur Berührungsstelle hin verjüngendem Querschnitt aufweist.

11. Bearbeitungsvorrichtung mit Pumpe nach einem der Ansprüche 8 bis 10, **dadurch gekennzeichnet, daß** die rückseitige Auflage (24) für die in Offenstellung befindliche Ventilscheibe (12) und/oder Ventilscheibenrückseite als linienförmige und/oder punktförmige Berührungsstellen eine Profilierung zur Verkleinerung der Anlageoberfläche aufweist.

12. Bearbeitungsvorrichtung mit Pumpe nach einem der Ansprüche 8 bis 11, **dadurch gekennzeichnet, daß** die rückseitige Auflage (24) für die Ventilscheibe (12) durch die Kanten eines treppenförmigen Vorsprunges oder mehrerer treppenförmiger Vorsprünge mit vorzugsweise linienförmigen Berührungsstellen gebildet sind.

## Claims

1. A process for evacuating a moist or liquid flow medium from the processing chamber (2) of a processing apparatus (1) by means of a conveying apparatus (3) which has a single- or multi-stage suction pump (4, 4'), wherein the flow medium is cooled during evacuation along its flow path in such a way that the flow medium in the conveying apparatus (3) is in, or is converted into, a liquid aggregate state, **characterised in that** the suction pump (4, 4') is constructed as a single- or multi-stage diaphragm pump and **in that** the cooling of the flow medium takes place at least in the area of at least one pump head (7) of the diaphragm pump (4, 4') and/or a connecting channel (27) which connects two pump stages (4a, 4b) of a multi-stage diaphragm pump (4') which are disposed in sequence in the direction of flow, and that the minimum of one pump head (7) and/or the connecting channel of the diaphragm pump (4, 4') is or are cooled to below the evaporation or boiling temperature that prevails under the given evacuation pressure.

2. A processing apparatus for implementing the process according to claim 1, with a processing chamber (2) which is attached to a conveying apparatus (3) for evacuation, and with a single or multi-stage suction pump (4, 4') associated with the conveying apparatus (3), wherein the conveying apparatus (3) can be cooled, at least in a partial area, by means of at least one cooling device (5), in such a way that the flow medium in the conveying apparatus (3) is in, or is converted into, a liquid state, **characterised in that** the suction pump (4, 4') is constructed as a single- or multi-stage diaphragm pump and at least one cooling device is provided to cool at least one pump head (7) of the diaphragm pump (4, 4') and/or a connecting channel (27) which connects two pump stages (4a, 4b) of a multi-stage diaphragm pump (4') disposed in sequence in the direction of flow, and **in that** the pump head (7) and/or the connecting channel (27) of the diaphragm pump (4, 4') can be cooled by this cooling device (5) in such a way that the head temperature of the diaphragm pump (4, 4') and/or the internal temperature of the connecting channel (27) are cooled to below the evaporation or boiling temperature that prevails under the given evacuation pressure.

3. A processing apparatus according to claim 2, **characterised in that** the cooling device (5) is configured as an air cooling system, preferably as a fan-assisted cooling system.

4. A processing apparatus according to claim 2 or 3, **characterised in that** the minimum of one pump head (7) disposed in the cooling stream of an air cooling system and/or the connecting channel (27) of the diaphragm pump (4, 4') has or have cooling fins (8) on its exterior in order to increase the cooling surface and optionally to guide the flow of the cooling stream.

5. A processing apparatus according to one of claims 2 to 4, **characterised in that** a relief valve is disposed in the flow guide provided between the processing chamber (2) and the outlet of the conveying apparatus (3).

6. A processing apparatus according to one of claims 2 to 5, **characterised in that** the relief valve is preferably the outlet valve (11) of the diaphragm pump (4, 4') or the outlet valve of one of its pump stages (4a, 4b) cooled in the area of the pump head.

7. A processing apparatus according to one of claims 2 to 6, **characterised in that** the processing apparatus is configured as a steam sterilisation apparatus (1) and its processing chamber is configured as a sterilising chamber (2).

8. A processing apparatus according to one of claims 2 to 7 with a single- or multi-stage diaphragm pump, with at least one inlet valve disposed in a pump head and at least one outlet valve, of which at least one valve has a valve disc (12) controlled by the flow medium which, in the closed position of the valve (10, 11), abuts in a sealing manner against a valve sealing surface (23) of the pump head (7) which defines at least one valve opening (22), **characterised in that** the sealing points of contact between the sealing side of the valve disc (12) and the valve sealing surface (23) of the pump head (7) which defines the valve opening (22) are substantially configured as linear points of contact.

9. A processing apparatus with a pump according to claim 8, **characterised in that** the points of contact between the rear side of the valve disc (12) and a seat (24) for the valve disc (12) disposed in its open position are substantially configured as linear or point-shaped points of contact.

10. A processing apparatus with a pump according to claim 8 and 9, **characterised in that** the valve sealing surface (23) and/or the sealing side of the valve disc (12) has at least one annular projection (26) which defines at least one valve opening (22), preferably with a cross-section which tapers conically towards the point of contact.

11. A processing apparatus with a pump according to one of claims 8 to 10, **characterised in that** the rearside seat (24) for the valve disc (12) and/or the rear side of the valve disc disposed in its open position, in the form of linear or point-shaped points of contact, has profiling to reduce the area of contact.

12. A processing apparatus with a pump according to one of claims 8 to 11, **characterised in that** the rearside seat (24) for the valve disc (12) is formed by the edges of a step-shaped projection or a plurality of step-shaped projections with preferably linear points of contact.

## Revendications

1. Procédé d'évacuation d'un fluide véhiculé humide ou liquide depuis la chambre de traitement (2) d'un dispositif de traitement (1) au moyen d'un dispositif de déplacement (3) qui présente une pompe aspirante à un ou plusieurs étages (4, 4'), le fluide véhiculé pendant l'évacuation étant refroidi au cours de la trajectoire d'écoulement de façon telle que le fluide véhiculé dans le dispositif de déplacement (3) se trouve ou est converti à l'état d'agrégat liquide, **caractérisé en ce que** la pompe aspirante (4, 4') est conçue en tant que pompe à membrane à un ou plusieurs étages, et le refroidissement du fluide véhiculé se fait au moins dans la zone d'au moins une tête de pompe (7) de la pompe à membrane (4, 4') et/ou d'un canal de liaison (27) reliant deux étages de pompe (4a, 4b) d'une pompe à membrane à plusieurs étages (4') qui se suivent dans la direction de l'écoulement, et **en ce que** la tête de pompe au moins (7) et/ou le canal de liaison de la pompe à membrane (4, 4') sont refroidis en-dessous de la température d'évaporation ou d'ébullition qui existe à une pression d'évacuation donnée.

2. Dispositif de traitement permettant de mettre en oeuvre le procédé selon la revendication 1, comportant une chambre de traitement (2), qui est reliée à un dispositif de déplacement (3) à des fins d'évacuation, ainsi qu'une pompe (4, 4') à un ou plusieurs étages qui est coordonnée au dispositif de déplacement (3), le dispositif de déplacement (3) pouvant être refroidi au moins dans une zone partielle au moyen d'au moins un dispositif de refroidissement (5) de façon telle que le fluide véhiculé dans le dispositif de déplacement (3) se trouve ou est converti à l'état d'agrégat liquide, **caractérisé en ce que** la pompe aspirante (4, 4') est conçue en tant que pompe à membrane à un ou plusieurs étages, et qu'est prévu au moins un dispositif de refroidissement destiné à refroidir au moins une tête de pompe (7) de la pompe à membrane (4, 4') et/ou un canal de liaison (27) reliant deux étages de pompe (4a, 4b) d'une pompe à membrane (4') à plusieurs étages qui suivent dans la direction de l'écoulement, et **en ce que** la tête de pompe (7) et/ou le canal de liaison (27) de la pompe à membrane (4, 4') peuvent être refroidis au moyen de ce dispositif de refroidissement (5) de façon telle que la température de tête de la pompe à membrane (4, 4') et/ou la température interne du canal de liaison (27) est refroidie en-dessous de la température d'évaporation ou d'ébullition qui existe à une pression d'évacuation donnée.

3. Dispositif de traitement selon la revendication 2, **caractérisé en ce que** le dispositif de refroidissement (5) est conçu en tant que refroidissement à air, de préférence en tant que refroidissement par ventilateur.

4. Dispositif de traitement selon la revendication 2 ou 3, **caractérisé en ce que** la tête de pompe (7) au moins disposée dans le flux de refroidissement d'un refroidissement à l'air et/ou le canal de liaison (27) de la pompe à membrane (4, 4') présente(nt) côté externe des nervures de refroidissement (8) destinées à augmenter la surface de refroidissement et éventuellement à diriger l'écoulement du flux de refroidissement.

5. Dispositif selon l'une des revendications 2 à 4, **caractérisé en ce qu'**est intercalée dans la direction de l'écoulement prévu entre la chambre de traitement (2) et la sortie du dispositif de déplacement (3) une soupape de retenue.

6. Dispositif de traitement selon l'une des revendications 2 à 5, **caractérisé en ce que** la soupape de retenue est de préférence la soupape de sortie (11) de la pompe à membrane (4, 4') ou la soupape de sortie d'un des étages de pompe (4a, 4b) refroidi dans la zone de la tête de pompe.

7. Dispositif de traitement selon l'une des revendications 2 à 6, **caractérisé en ce que** le dispositif de traitement est conçu en tant que dispositif de stérilisation à la vapeur (1) et sa chambre de traitement en tant que chambre de stérilisation (2).

8. Dispositif de traitement selon l'une des revendications 2 à 7, équipé d'une pompe à membrane à un ou plusieurs étages, d'au moins une soupape d'entrée se trouvant dans une tête de pompe et d'au moins une soupape de sortie, parmi lesquelles au moins une soupape présente un disque de soupape (12) commandé par le fluide véhiculé, qui, dans la position de fermeture de la soupape (10, 11), adhère de façon étanche contre une portée de siège de soupape (23) de la tête de pompe (7) entourant au moins une ouverture de soupape (22), **caractérisé en ce que** les points de contact étanchéifiants entre le côté d'étanchéité du disque de soupape (12) et la portée de siège de soupape (23) entourant l'ouverture de soupape (22) de la tête de pompe (7) sont essentiellement conçus en tant que points de contact en forme de ligne.

9. Dispositif de traitement présentant une pompe selon la revendication 8, **caractérisé en ce que** les points de contact entre le côté arrière du disque de soupape (12) et un appui (24) pour le disque de soupape (12) se trouvant dans la position ouverte sont conçus essentiellement en tant que points de contact en forme de lignes et/ou de points.

10. Dispositif de traitement présentant une pompe selon les revendications 8 et 9, **caractérisé en ce que** la portée de siège de soupape (23) et/ou le côté d'étanchéité du disque de soupape (12) présente(nt) au moins une saillie annulaire (26) entourant une ouverture de soupape (22) et possédant une section transversale de préférence conique qui se rétrécit vers les points de contact.

11. Dispositif de traitement présentant une pompe selon l'une des revendications 8 à 10, **caractérisé en ce que** l'appui arrière (24) pour le disque de soupape (12) se trouvant en position ouverte et/ou l'arrière du disque de soupape présente(nt) en tant que points de contact en forme de ligne et/ou en forme de point un profilage de rétrécissement de la surface de contact.

12. Dispositif de traitement présentant une pompe selon l'une des revendications 8 à 11, **caractérisé en ce que** l'appui arrière (24) pour le disque de soupape (12) est formé par les arêtes d'une saillie en forme d'escalier ou de plusieurs saillies en forme d'escalier, présentant de préférence des points de contact en forme de ligne.
